# EUROPEAN PATENT SPECIFICATION

(11) **EP 1 347 805 B1**
(45) Date of publication and mention of the grant of the patent: **29.07.2009**
(21) Application number: 01986916.3
(22) Date of filing: 20.12.2001
(51) Int. Cl.: A61P 31/00

(54) **PHARMACEUTICAL PREPARATION FOR DOUCHES AND/OR IRRIGATIONS OF NATURAL OR PATHOLOGICAL CAVITIES IN THE HUMAN BODY**
PHARMAZEUTISCHE ZUBEREITUNG ZUR SPÜLUNG UND/ODER ZUM BESPRÜHEN VON NATURLICHEN ODER PATHOLOGISCHEN KÖRPERHÖHLEN IM MENSCHLICHEN KÖRPER
PREPARATION POUR LAVAGES INTERNES ET/OU IRRIGATIONS DE CAVITES NATURELLES OU PATHOLOGIQUES DU CORPS HUMAIN

(30) Priority: 04.01.2001 IT MO20010002
(43) Date of publication of application: 01.10.2003
(73) Proprietor: Palmieri, Beniamino, I-41100 Modena (IT)
(72) Inventor: Palmieri, Beniamino, I-41100 Modena (IT)
(74) Representative: Minoja, Fabrizio
(86) International application number: PCT/EP2001/015114
(87) International publication number: WO 2002/053110

(56) References cited:
- EP-A- 0 414 373
- EP-A- 1 020 180
- WO-A-00/27339
- RU-C- 2 111 741
- US-A- 4 682 979
- US-A- 4 886 786
- US-A- 5 181 908
- US-A- 5 735 833
- US-A- 6 113 922
- PATENT ABSTRACTS OF JAPAN vol. 2000, no. 13, 5 February 2001 (2001-02-05) & JP 2000 281147 A (OTSUKA PHARMACEUT FACTORY INC;SENJU PHARMACEUT CO LTD), 10 October 2000 (2000-10-10)
- DATABASE WPI Section PQ, Week 198328 Derwent Publications Ltd., London, GB; Class P33, AN 1983-710146 XP002208452 & SU 955 922 A (CHERNTSAVANSK SOYUZ), 7 September 1982 (1982-09-07)
- DATABASE WPI Section PQ, Week 199234 Derwent Publications Ltd., London, GB; Class P31, AN 1992-282699 XP002208453 & SU 1 690 701 A (TASHK MED INST), 15 November 1991 (1991-11-15)
- PATENT ABSTRACTS OF JAPAN vol. 2002, no. 04, 4 August 2002 (2002-08-04) & JP 2001 354549 A (KOIKE KAGAKU KK), 25 December 2001 (2001-12-25)

## Description

The present invention relates to a preparation for douches and/or irrigations of natural or pathological cavities in the human body, and is designed for pre-, intra- and/or post-operative use, for example for hydrodissection, debridement and irrigation of arteriotomies, brain irrigations, irrigations of bloody parenchymal surfaces, etc.

In accordance with the prior art, preparations are known which are designed for douches and/or irrigations of natural or pathological cavities in the human body and contain active constituents suitable for debridement, deodorisation, disinfection, desensitisation, etc., in the form of liquids, foams, gels, etc.

The use of these active constituents always involves the risk of allergic reactions or local hypersensitivity in the patient. In addition, said preparations are not always effective when it is necessary to remove bacterial agglomerates, filaments of mucous or mucopus, etc., from said cavities.
- D1 discloses an eye perfusion and a washing liquid bag consisting of two different chambers, one containing oxyglutathione and the other sodium bicarbonate, said bags being included into a packaging filled with carbon dioxide gas.
- D2 discloses compositions for improving healing of wounds consisting of water solution comprising carbon dioxide which must be present in the solution always in the dissolved state.
- D3 discloses a visco-elastic formulation of a hyaluronate salt for ophthalmic surgery optionally containing bicarbonate.
- D4 discloses injectable aqueous solutions for use in ultrasound surgery, particularly in ultrasound-assisted in lipoplasty. Carbon dioxide or other gases are added to the solutions in order to reduce or limit "cavitation" generated by ultrasound application. "
- D5 discloses intraocular irrigation solution comprising a glycosaminoglycan .
- D6 discloses an oral lavage for use as preoperative gastro-intestinal surgery. Said lavage contains polyethylene glycol and a number of salts.
- D7 discloses a colon washing apparatus injecting under pressure water into the colon of a patient. The possibility of introducing a gentle flow of oxygen or compressed air into the water stream is envisaged during the apparatus operation.
- D8 discloses an apparatus for cleansing living tissue which includes a source of sterile liquid and a source of pressurized gas, particularly oxygen, providing a sterile liquid mist in a high velocity gas stream.
- D9 discloses a lavage tip including an oxygen port to provide oxygen therapy to promote faster healing.
- D10 discloses a suction catheter for delivering an oxygenated irrigating fluid.
- D11 discloses an apparatus for oxygen-aero hydromassage for the oral cavity.
- D12 discloses oxygenated polyion solutions comprising antibiotics for the treatment of peritonitis.

The purpose of the present invention is to provide a preparation for douches and/or irrigations of natural or pathological cavities in the human body which has an effective cleansing and bacteriostatic action and does not trigger, or at least minimises the risk of allergic reactions or local hypersensitivity.

The present invention relates to a preparation for douches and/or irrigations of natural or pathological cavities in the human body, **characterised in that** it comprises water to which carbon dioxide is added, optionally in combination with oxygen, nitrogen, gaseous anaesthetic substances and inert gases.

Carbon dioxide allows the pH of the preparation to be adjusted in a simple, controllable way to adapt it to the particular type of natural or pathological cavity to be cleansed or irrigated, because the pH of the preparation varies when the quantity of carbon dioxide dissolved in the water is varied.

Oxygen, added in a quantity which does not cause oxidative phenomena or tissue damages, optimises the therapeutic effects of the preparation of the invention.

Moreover, carbon dioxide, which develops in the form of bubbles when the preparation is introduced into said cavities, performs a bacteriostatic action, in particular against microaerophilic and aerobic germs.

The development of said carbon dioxide in the form of bubbles gives rise to a mechanical action which facilitates the removal of bacterial agglomerates, filaments of mucous, mucopus etc. from the interior of said cavities.

Furthermore, carbon dioxide stimulates hyperaemia of the surface capillaries of the walls of said cavities, activating the trophism of the mucosal walls, and facilitates the normalisation of antibody responses.

In particular, in irrigations performed during laparoscopic surgery, when carbon dioxide is insufflated into the patient's abdomen, the use of the preparation of the invention enables the required carbon dioxide concentration to be maintained in the patient's abdomen during the irrigation stage. Moreover, carbon dioxide can be bubbled, together with other active constituents, over the entire peritoneal surface in order to facilitate the diffusion of said active constituents and obtain particular pharmacological effects, such as macrophagic activation of the mesothelial cells, stimulation of fibrinolytic effects to prevent the formation of adhesions, etc.

The preparation of the invention may include mineral water with a variable concentration of carbon dioxide, preferably ranging between approximately 1% and approximately 30%, with a preferred concentration of approximately 10%, and/or an oxygen concentration of up to 300-600 mg/litre.

The carbon dioxide can be added to the water by industrial processes of known type or can be performed in an extempore manner, for example by mixing citric acid and potassium bicarbonate in equal parts, diluted in water and lactic acid.

Oxygen can be added by bubbling under pressure, sonication, or other known chemical or physical methods of dissolving oxygen in water.

The preparation may also include substances with a cleansing, deodorising, desensitising, anti-itching, anti-inflammatory, wound-healing, revulsive, eudermic and reparatory, antibacterial, antiparasitic, antifungal, antiviral, antiexudative, corticosteroid, antitumoral, immunomodulating, haemostatic, haemorheological and hormonal activity.

Said substances should preferably have a formulation which does not form precipitates or insoluble salts on reacting with the carbon dioxide dissolved in the water, thus preventing its development in the form of bubbles. The use of substances that form precipitates or insoluble salts on reacting with carbon dioxide is acceptable if their concentration or formulation does not significantly reduce the development of carbon dioxide in bubble form during the use of the preparation of the invention.

The preparation of the invention could also contain bacteria, such as lactobacilli or other carbon-dioxide-producing bacteria, which are able to live in an acid environment and restore the bacterial flora in natural body cavities suffering from damage caused by dysmicrobism, such as the vagina, colorectum, urethra, bladder, etc.

The preparation of the invention can be advantageously used to aid penetration of genetic material into the cells, such as carriers or viruses, thus facilitating the transmembranal passage of the material thanks to the carbon dioxide dissolved in the water, which aids the passage of said genetic material through the cell membranes. Similarly, the action of carbon dioxide facilitates the absorption by the cells of antitumoral chemotherapy substances, thus making their action faster and more effective.

The use of the preparation of the invention is particularly advantageous for pre-, intra, and post-operative irrigations of the bladder cavity and/or the prostatic bed and/or the renal pelvis after prostatectomy, in view of its ability to aid the removal of fibrin fragments, inflammatory cells, mucous etc. as a result of the mechanical action exerted by the development of carbon dioxide bubbles. The use of the preparation of the invention for irrigations of the renal pelvis and urethral duct is also particularly advantageous for patients with indwelling catheters and for the hygiene of the urethral meatus, the male external genitals and the female urethra, especially in cases of chronic bacterial, fungal or vital infections.

In the female genital apparatus, the use of the preparation of the invention to irrigate the clitoris can accentuate the sensitivity and congestion of that anatomical structure, thus curing symptoms leading to frigidity and dyspareunia. The use of the preparation of the invention for bladder irrigations is particularly advantageous in case of cystitis with frequent, urgent urination, because the stimulation of hyperaemia of the capillaries on the bladder surface performed by carbon dioxide aids the elimination of toxic waste and the penetration into that wall of long-acting desensitising or anaesthetising active constituents which can be added to the preparation of the invention.

The hyperaemic action of carbon dioxide is advantageous in all cases requiring penetration of active constituents such as intravescical instillations of chemotherapeutics or BCG (tuberculin bacillus extract).

The hyperaemisation effect can be regulated by heating or cooling the preparation of the invention before introducing it into the cavity to be washed or irrigated.

The preparation of the invention can also be used for aerosol, for lavages after disobstruction of the auditory duct, the fallopian tubes, the spermatic ducts in general, for lavages after surgical disobstruction or for dilating arterias and facilitate their debridment.

More particularly, when the preparation of the invention contains large amounts of dissolved carbon dioxide, can include active constituents to make a parenteral perfusion solution, which can also be used as a dialysis solution for the peritoneal, pleural, pericardial or cranial joint cavity, to aid the removal of toxic solutes.

The preparation of the invention can be used to prepare evacuation and cleansing enemas before surgery and endoscopic procedures, or in patients suffering from intestinal diverticuli, stenosis or antony, ulcerous colitis or Crohn's disease, to alleviate the symptoms, eliminate bacteria and exudative and excrementitious concretions, and facilitate penetration of active constituents and lymph absorption.

The preparation of the invention can be used to obtain anaesthetic effects if the gas dissolved in the water is an anaesthetic gas such as nitrous oxide.

The preparation of the invention can be administered with a rubber syringe or a squeeze bottle, optionally fitted with a spray device or a metering valve, or a portable variable preparation apparatus.

In practice, the composition of the preparation of the invention can differ from that indicated, while still remaining within the legal protection of the invention.

## Claims

1. The use of water containing carbon dioxide developing in the form of bubbles for the preparation of douches and/or irrigations having bacteriostatic, cleansing and hyperhaemic action on natural or pathological cavities of the human body.

## Patentansprüche

1. Verwendung von Wasser, welches Kohlendioxid umfasst, wobei das Kohlendioxid in der Form von Blasen gebildet wird, für die Verstellung von Duschen und/oder Spülungen mit bakteriostatischer, reinigender und hyperämischer Wirkung auf natürliche oder pathologische Kavitäten des menschlichen Körpers.

## Revendications

1. Utilisation d'eau contenant du dioxyde de carbone se développant sous forme de bulles pour la préparation de douches et/ou d'irrigations ayant une action bactériostatique, nettoyante et hyperhémique sur les cavités naturelles ou pathologiques du corps humain.
